# EUROPÄISCHE PATENTSCHRIFT

(11) **EP 2 151 231 B1**
(45) Veröffentlichungstag und Bekanntmachung des Hinweises auf die Patenterteilung: **02.08.2017**
(21) Anmeldenummer: 08161854.8
(22) Anmeldetag: 05.08.2008
(51) Int. Cl.: A61K 8/31, A61Q 19/08

(54) **Kosmetische Formulierung**
Cosmetic formulation
Formule cosmétique

(43) Veröffentlichungstag der Anmeldung: 10.02.2010
(73) Patentinhaber: PM-International AG, 5445 Schengen (LU)
(72) Erfinder: Meinhardt, Horst, 56249 Herschbach (DE); Sorg, Rolf, 5444 Schengen (LU)
(74) Vertreter: Zech, Stefan Markus

(56) Entgegenhaltungen:
- DE-A1- 19 609 477
- US-A1- 2005 042 233
- US-A1- 2007 065 396
- US-B1- 6 383 474
- US-B1- 6 605 298

## Beschreibung

Die Erfindung betrifft eine kosmetische Formulierung zur dermatologischen Anwendung.

Um einen optimalen oxidativen Schutz der Haut zu erreichen, ist es notwendig entsprechende Wirkstoffe in assimilierbarer Form in kosmetischen Produkten einzusetzen bzw. die kosmetischen Produkte selbst möglichst optimal zu formulieren.

Dabei werden als antioxidative Wirkstoffe in der Kosmetik beispielsweise Vitamin E, Vitamin C und viele weitere sogenannte Antioxidantien eingesetzt.

Eine besondere Klasse der antioxidativen Stoffe stellen die Karotinoide dar. Die bisher genutzten Karotinoide haben jedoch durch ihre intensive Färbung in der Kosmetik nur einen begrenzten Einsatzbereich. Insbesondere die Anfärbung der Haut, aber auch die Anfärbung der Kleidung wird dabei als unangenehme Begleiterscheinung des Einsatzes dieser Karotinoide in der Hautpflege angesehen.

Die EP 1 107 723 B1 sowie die US 6 383 474 B1 beschreiben ein Karotinoid enthaltendes Präparat, welches in Nahrung, in der Kosmetik und in einer Vielzahl von medizinischen Anwendungen eingesetzt werden kann. Die beschriebenen Karotinoide, nämlich Phytoen (7, 8, 11, 12, 7', 8', 11', 12' - Oktahydro-y, γ-Karotin) und Phytofluen (15Z, 7, 8, 11, 12, 7', 8' - Exahydro-γ, γ-Karotin) weisen antioxidative Eigenschaften auf und sind zudem noch geeignet ultraviolettes (UV) Licht zu absorbieren. Außerdem sind Phytoen und Phytofluen viel stabiler gegenüber Oxidation als beispielsweise Beta-Karotin. Die Entdeckung dieser Eigenschaften führte zu der Erkenntnis, dass Phytoen und Phytofluen in Kombination besonders wirkungsvoll Schäden, verursacht durch verschiedenste Umwelteinflüsse, vorbeugt.

In der bereits erwähnten EP 1 107 723 B1 wird ein Herstellungsverfahren für Phytoen und Phytofluen beschrieben. Dabei werden verschiedene Karotinoid produzierende Organismen, wie verschiedenste Pflanzen, verschiedene Algen, und besonders die Mikro-Alge Dunaliella SP genutzt. Die Karotinoid produzierenden Organismen werden beispielsweise im Fall der Dunaliella SP in Außen-Pools in einem Meerwasser bzw. Salzwasser enthaltenden Medium bei einer Temperatur zwischen 10°C und 40°C gehalten.

Die färbende Wirkung der genannten Karotinoide ist gegenüber herkömmlichen Karotinoiden bereits deutlich vermindert; so ist beispielsweise das Phytoen an sich farblos und das Phytofluen nur schwach färbend. Dies führt dazu, dass nach einem Auftragen von Phytoen und Phytofluen auf die Haut, eine Anfärbung dieser deutlich reduziert ist, wobei eine geringe Anfärbung der Haut dennoch nicht gänzlich vermieden wird. Außerdem kann durch den Einsatz dieser Karotinoide zwar ebenfalls eine Anfärbung der Kleidung verringert werden, wobei aber das an sich farblose Phytoen sowie das schwachfärbende Phytofluen - ähnlich wie beispielsweise ein farbloses Öl - die optischen Eigenschaften der Kleidung auf als unangenehm empfundene Art und Weise verändern.

Die Aufgabe der vorliegenden Erfindung besteht darin, eine Karotinoid enthaltende kosmetische Formulierung zur dermatologischen Anwendung bereitzustellen, die keine merkliche Anfärbung der Haut zur Folge hat und insbesondere eine optische Beeinträchtigung der Kleidung gegenüber dem Stand der Technik weiter verringert.

Diese Aufgabe wird gelöst durch eine kosmetische Formulierung nach den Merkmalen des Anspruches 1 sowie durch ein Verfahren zur Herstellung der Formulierung nach Anspruch 9, 10 oder 12. Vorteilhafte Weiterbildungen sind in den Unteransprüchen angegeben.

Die kosmetische Formulierung zur dermatologischen Anwendung enthält mindestens ein Karotinoid, wobei das mindestens eine Karotinoid bei der dermatologischen Anwendung im Wesentlichen farblos ist und/oder mit mindestens einem weiteren antioxidativen Wirkstoff - insbesondere einem weiteren Karotinoid - eine Kombination bildet, die bei der dermatologischen Anwendung im Wesentlichen farblos ist und wobei das mindestens eine Karotinoid in nanomizellierter Form vorliegt. Konkret liegt als Karotinoid Phytoen und/oder Phytofluen vor. "Im Wesentlichen farblos" soll an dieser Stelle dahingehend verstanden werden, dass eine Anfärbung bzw. optische Beeinträchtigung (beispielsweise eine Veränderung der Helligkeit oder Farbintensität) der Haut und der Kleidung bei der dermatologischen Anwendung für das menschliche Auge nicht erkennbar sein soll. Das nanomizellierte Karotinoid kann beispielsweise in unvermischter Form vorliegen oder in eine lipophile Matrix eingebunden sein. Phytoen ist bereits in nicht-nanomizellierter Form farblos und daher besonders für den Einsatz in der kosmetischen Formulierung geeignet. In ähnlicher Weise zeichnet sich Phytofluen selbst in nicht-nanomizellierter Form nur durch eine geringe färbende Wirkung aus, so dass auch der Einsatz von Phytofluen zu bevorzugen ist.

Das Verfahren zur Herstellung der Formulierung umfasst die Schritte: a) Bereitstellen einer Mischung aus mindestens einem Polyol, aus mindestens einem Phospholipid, aus Squalan und mindestens einem farblosen Karotinoid, wobei als Karotinoid Phytoen und/oder Phytofluen zum Einsatz kommen. b) Dispergieren dieser Mischung durch Rühren und c) Weiterverarbeiten der Mischung durch Hochdruckhomogenisation zu einer transparenten Mischung.

Ein wesentlicher Aspekt der Erfindung liegt also darin, dass das/die zum Einsatz kommende(n) Karotinoid(e) in nanomizellierter Form, das heißt in mizellierter Form, bei der die durchschnittliche Mizellen-Größe einen Wert von 100 nm nicht überschreitet, vorliegen. Eine solche Nano-Mizellierung weist folgende Vorteile gegenüber dem Stand der Technik auf.

Die kleine Mizellen-Größe führt dazu, dass die Karotinoide transparent oder zumindest opak erscheinen und außerdem besonders tief in die Haut eindringen. Die beiden letztgenannten Vorteile, nämlich die Penetration in tiefere Hautschichten sowie die erhöhte Transparenz bzw. Opazität der Nano-Mizellen führt dazu, dass selbst bei der Verwendung von an sich stärker gefärbten Karotinoiden jene Karotinoide zumindest bei der dermatologischen Anwendung im Wesentlichen farblos sind; wobei, wie bereits erwähnt, mit "farblos" hier die Eigenschaft der nanomizellierten Karotinoide gemeint ist, bei der dermatologischen Anwendung nicht zu einer Verfärbung oder anderweitigen optischen Veränderung der Haut und der Kleidung zu führen.

Durch das Eindringen in tiefere Hautschichten, wird also insbesondere das Risiko einer Verfärbung, Helligkeitsänderung oder Farbintensitätsänderung der Kleidung während der dermatologischen Anwendung deutlich reduziert.

Weiterhin wird durch die mit der Nanomizellierung verbundene verbesserte Penetration in tiefere Hautschichten erreicht, dass die antioxidative Wirkung der Karotinoide besonders gut ausgenutzt wird. Noch ein weiterer Vorteil der Nanomizellenstruktur besteht darin, dass dadurch die entsprechenden Karotinoide besser geschützt werden, was wiederum deren Haltbarkeit verlängert.

Die kosmetische Formulierung umfasst weiterhin mindestens eine wasserlösliche, antioxidative Aminosäure. Insbesondere für den Fall, dass jene zusätzliche wasserlösliche, antioxidative Aminosäure nicht in mizellierter bzw. nano-mizellierter Form vorliegt, wird ein tieferes Eindringen jener Komponenten durch eine Art "Mitreißeffekt" bewirkt. Dadurch kann die wasserlösliche antioxidative Komponente wesentlich effektiver, insbesondere schneller und/oder tiefer innerhalb der Haut resorbiert werden.

Vorzugsweise wird mindestens eine der wasserlöslichen Aminosäuren durch Thiotain (INCI: Ergothionein) gebildet. Thiotain ist eine multifunktionelle Aminosäure. Diese körpereigene Substanz - nachweisbar als OCTN-1-Transporter in dermalen Fibroblasten - ist ein äußerst stabiles, farb- und geruchloses Antioxidanz, das Ubichinon sowie Idebenon als Radikalfänger deutlich überlegen ist. Durch eine solche Kombination von Thiotain mit den farblosen, nanomizellierten Karotinoiden entstehen hautpflegende und hautschützende Produkte mit überlegener antioxidativer Wirkung und eleganter Anmutung ohne Färbungs- oder Stabilitätsprobleme.

Vorzugsweise umfasst die kosmetische Formulierung weiterhin Polyol, insbesondere Glyzerin und/oder wenigstens ein Phospholipid und/oder Squalan, wobei das Verhältnis Polyol (zum Beispiel Glyzerin) : Phospholipid bevorzugtermaßen zwischen 100 : 1 und 1 : 1, weiter vorzugsweise zwischen 30 : 1 und 5 : 1, insbesondere bei etwa 15 : 1 liegt und wobei das Verhältnis Squalan : farblose(s) Karotinoid(e) bevorzugtermaßen zwischen 10 : 1 und 1 : 10, weiter vorzugsweise zwischen 5 : 1 und 1 : 5, insbesondere etwa 1 : 1 beträgt. Vorzugsweise weist/weisen das/die Phospholipid(e) einen Phosphatidylcholin-Gehalt (PC-Gehalt) zwischen 50% und 90%, insbesondere zwischen 65% und 75% auf.

Ein alternatives Verfahren zur Herstellung der Formulierung umfasst die Schritte: a) Vermischen von Squalan und farblosem Karotinoid, wobei als Karotinoid Phytoen und/oder Phytofluen zum Einsatz kommen, mit hydrophobiertem Inulin, b) Herstellung einer Voremulsion durch Vermischen des Squalan, des/der farblosen Karotinoids/Karotinoide und des hydrophobierten Inulins mit Wasser und Polyol mittels Rotor-Stator-Homogenisation und c) Weiterverarbeiten der Voremulsion mittels Hochdruckhomogenisation.

Vorzugsweise wird Sonnenblumenöl und/oder anderer Triglyzeride enthaltende Öle und/oder Jojobaöl und/oder Sheabutter als Polyol verwendet.

Als zusätzlicher Schritt d) wird eine wässrige, farblose Aminosäure (zum Beispiel Thiotain) unter Rühren zugesetzt.

Weitere Ausführungsformen ergeben sich aus den Unteransprüchen.

Nachfolgend wird die Erfindung auch hinsichtlich weiterer Merkmale und Vorteile anhand von Ausführungsbeispielen beschrieben.

### Beispiel 1:

Phospholipide, dispergiert in hochkonzentrierten wässrigen Lösungen von Polyolen oder Kohlenhydraten, sind in der Lage, mit farblosen Karotinoiden (wie beispielsweise Phytoen oder Phytofluen) in einer lipophilen Matrix Emulsionen zu bilden, die aufgrund der extrem kleinen Größe der Nanomizellen (30 nm bis 60 nm) transparent bzw. durchscheinend sind. Durch die extrem kleine Größe der Nanomizellen ist eine optimale Penetration in tiefere Hautschichten gewährleistet, wo die antioxidative Wirkung der Karotinoide von größerem Nutzen ist und insbesondere der Effekt der Anfärbung der äußeren Hautoberfläche bzw. hiervon ausgehend der Kleidungsstücke in entscheidendem maße weiter reduziert wird.

In Kombination mit der wasserlöslichen Aminosäure Thiotain wird das Wirkspektrum erweitert und ein neuartiger Wirksynergismus erzeugt.

In einer bevorzugten Weiterbildung des Verfahrens wird als zusätzlicher Schritt d) eine wässrige, farblose Aminosäure (zum Beispiel Thiotain) unter Rühren zugesetzt.

Weitere Ausführungsformen ergeben sich aus den Unteransprüchen.

Nachfolgend wird die Erfindung auch hinsichtlich weiterer Merkmale und Vorteile anhand von Ausführungsbeispielen beschrieben.

### Beispiel 1:

Phospholipide, dispergiert in hochkonzentrierten wässrigen Lösungen von Polyolen oder Kohlenhydraten, sind in der Lage, mit farblosen Karotinoiden (wie beispielsweise Phytoen oder Phytofluen) in einer lipophilen Matrix Emulsionen zu bilden, die aufgrund der extrem kleinen Größe der Nanomizellen (30 nm bis 60 nm) transparent bzw. durchscheinend sind. Durch die extrem kleine Größe der Nanomizellen ist eine optimale Penetration in tiefere Hautschichten gewährleistet, wo die antioxidative Wirkung der Karotinoide von größerem Nutzen ist und insbesondere der Effekt der Anfärbung der äußeren Hautoberfläche bzw. hiervon ausgehend der Kleidungsstücke in entscheidendem maße weiter reduziert wird.

In Kombination mit der wasserlöslichen Aminosäure Thiotain wird das Wirkspektrum erweitert und ein neuartiger Wirksynergismus erzeugt.

Darüber hinaus wird eine bisher nicht bekannte Stabilität und Farblosigkeit gegenüber Produkten auf herkömmlicher Basis erreicht.

Die folgenden Beispiele 2 bis 6 beziehen sich auf konkrete Verfahren zur Herstellung der nanomizellierte Karotinoide enthaltenden Formulierungen.

### Beispiel 2:

In 7,5 kg Glyzerin werden 500g Phospholipid mit einem PC-Gehalt (Phosphatidylcholin-Gehalt) von ca. 70% zugefügt sowie 2 kg einer Mischung Squalan mit ca. 2 g farblosen Karotinoiden (wie beispielsweise Phytoen). Diese Mischung wird durch Rühren dispergiert und dann durch Hochdruckhomogenisation zu einer transparenten Mischung mit einer Teilchengröße von ca. 30 nm bis 60 nm weiterverarbeitet.

Diese Mischung kann direkt oder als Verdünnung auf der Haut eingesetzt werden. Die beschriebene Mischung kann auch in verschiedene Körperpflege-Formulierungen eingearbeitet werden, beispielsweise in O/W-Cremes (Öl-in-Wasser-Cremes) oder O/W-Lotionen oder Sprays, W/O-Cremes (Wassertröpfchen-in-Öl-Cremes) oder -Lotionen, gel-artige Formulierungen oder fetthaltige Formulierungen.

### Beispiel 3:

In 7,5 kg Glyzerin werden 500 g Phospholipid mit einem PC-Gehalt von ca. 70% sowie 2 kg einer Mischung Squalan mit ca. 2 g farblosen Karotinoiden zugefügt. Diese Mischung wird durch Rühren dispergiert und dann durch Hochdruckhomogenisation zu einer transparenten Mischung mit einer Teilchengröße von ca. 30 nm bis 60 nm weiterverarbeitet. Dieser so entstandenen nanomizelliertes Karotinoid enthaltenden Emulsion werden 100 g einer wässrigen Lösung mit 0,1 % bis 1% einer wässrigen, farblosen Aminosäure (beispielsweise Thiotain) unter Rühren zugesetzt. Alternativ kann die vorgenannte wässrige Lösung auch mit der nanomizelliertes Karotinoid enthaltenden Emulsion dem kosmetischen Endprodukt ohne vorherige separate Vermischung zugegeben werden.

### Beispiel 4:

2 kg einer Mischung Squalan mit ca. 2 g nicht-färbenden Karotinoiden werden mit 200 g hydrophobiertem Inulin (Inulin Lauryl Carbamat) gemischt und dann in einer Mischung von 7,3 kg Wasser und 500 g Glyzerin durch den Einsatz einer Rotor-Stator-Homogenisation zu einer Voremulsion verarbeitet. Diese Voremulsion wird dann mit einer Hochdruckhomogenisation bei 500 bis 700 bar zu einer nanomizelliertes Karotinoid enthaltenden Emulsion mit einer Nanomizellengröße von ca. 50 nm bis 100 nm verarbeitet. Diese Mischung kann in gleicher Weise wie in Beispiel 2 beschrieben eingesetzt werden.

### Beispiel 5:

1 kg Sonnenblumen- und/oder andere Triglyzeride und 500 g Jojobaöl und 300 g Sheabutter und 200 g einer Mischung Squalan mit ca. 200 mg nicht-färbenden Karotinoiden werden mit 200 g hydrophobiertem Inulin (Inulin Lauryl Carbamat) gemischt und dann in einer Mischung von 7,3 kg Wasser und 500 g Glyzerin und 500 g Pentylenglykol durch den Einsatz einer Rotor-Stator-Homogenisation zu einer Voremulsion verarbeitet. Diese Voremulsion wird dann mit einer Hochdruckhomogenisation bei 500 bis 700 bar zu einer nanomizelliertes Karotinoid enthaltenden Emulsion mit einer Nanomizellengröße von ca. 50 nm bis 100 nm verarbeitet. Dieser so entstandenen Emulsion werden 50 g einer wässrigen Lösung mit 0,1 % bis 0,5 % einer wässrigen-farblosen Aminosäure (beispielsweise Thiotain) unter Rühren zugesetzt.

Diese Mischung kann direkt oder als Verdünnung auf der Haut als sprühbare Emulsion eingesetzt bzw. alternativ, wie unter Beispiel 2 beschrieben, verwendet werden.

### Beispiel 6:

In 400 g entionisiertes Wasser werden 100 g Glykosyl Ceramide unter Rühren dispergiert und hydratisiert. Diese Dispersion wird unter weiterem Rühren auf 50°C bis 70 °C, vorzugsweise etwa 60°C erwärmt bis eine homogene Masse entsteht.

Unter weiterem Rühren werden dann 50 g einer Mischung von Squalan und farblosen Karotinoiden in die Masse eingerührt und mit einem Rotor-Stator-System 8 bis 12 Minuten, vorzugsweise etwa 10 Minuten homogenisiert bis die gewünschte Nanomizellengröße von ca. 100 bis 150 nm erreicht ist.

Die Homogenisierung kann auch mit anderen Verfahren durchgeführt werden (z.B. Hochdruckhomogenisierung oder Ultraschallhomogenisierung), um eine noch kleiner Mizellengröße zu erreichen.

Das so entstandene Produkt kann als Pflegeprodukt direkt auf der Haut angewendet werden und/oder als verdünnte, wässerige Mischung oder als Zusatz in Hautpflegeprodukten eingesetzt werden.

Alternativ kann in Beispiel 6 anstatt 100 g Glykosyl Ceramiden eine Mischung von 100 g Glykosyl Ceramiden und Digalactosyl Diglyzeriden eingesetzt werden.

### Beispiel 7:

In 400 g entionisiertes Wasser werden 100 g Glykosyl Ceramide unter Rühren dispergiert und hydratisiert. Diese Dispersion wird unter weiterem Rühren auf 50°C bis 70 °C, vorzugsweise etwa 60°C erwärmt bis eine homogene Masse entsteht.

Unter weiterem Rühren werden dann 50 g einer Mischung von Squalan und farblosen Karotinoiden in die Masse eingerührt und mit einem Rotor-Stator-System 8 bis 12 Minuten, vorzugsweise etwa 10 Minuten homogenisiert bis die gewünschte Nanomizellengrößegröße von ca. 100 bis 150 nm erreicht ist.

Diese mizellierte Mischung wird dann auf 40° gekühlt, anschließend Thiotain zugeführt und schließlich eine weitere Homogenisierung zur Mizellierung der Aminosäure durchgeführt. Alternativ kann die Aminosäure in Form von Thiotain aber auch bereits schon vor Zugabe des Karotinoids zugeführt werden.

Auch hier kann die Homogenisierung mit anderen Verfahren bewerkstelligt werden (z. B. Hochdruckhomogenisierung oder Ultraschallhomogenisierung), um eine noch kleinere Mizellengröße zu erreichen. Das so entstandene Produkt kann auch hier als Pflegeprodukt direkt auf der Haut angewendet werden und/oder als verdünnte, wässrige Mischung oder als Zusatz in Hautpflegeprodukten eingesetzt werden. Auch hier kann alternativ, entsprechend Beispiel 6, anstatt 100 g Glykosyl Ceramiden eine Mischung von 100 g Glykosyl Ceramiden und Digalactosyl-Diglyceriden eingesetzt werden.

Die in den Beispielen 1 - 7 beschriebenen farblosen Karotinoide können beispielsweise über das in der EP 1 107 723 B1 beschriebene Verfahren hergestellt werden. Die jeweiligen Mengen- und Parameterangaben der Beispiele 1-7 können variieren. Die Verfahren nach den Beispielen 2-7 können kombiniert werden, wobei insbesondere einzelne Verfahrensschritte austauschbar sind.

## Patentansprüche

1. Kosmetische Formulierung zur dermatologischen Anwendung, enthaltend mindestens ein Karotinoid, wobei
- als Karotinoid Phytoen und/oder Phytofluen vorliegt und wobei
- das Phytoen und/oder Phytofluen in nanomizellierter Form vorliegt,
und mindestens eine wasserlösliche, antioxidative Aminosäure, wobei die kosmetische Formulierung Glycosyl-Ceramide und/oder Digalactosyl-Diglyzeride umfasst.

2. Kosmetische Formulierung nach Anspruch 1,
wobei mindestens eine wasserlösliche Aminosäure Ergothionein ist.

3. Kosmetische Formulierung nach Anspruch 1 oder 2,
**dadurch gekennzeichnet, dass**
die wasserlösliche antioxidative Aminosäure in die Nanomizellierung der Karotinoide mit einbezogen ist.

4. Kosmetische Formulierung nach einem der vorangehenden Ansprüche,
**dadurch gekennzeichnet, dass**
die kosmetische Formulierung weiterhin Polyol, insbesondere Glyzerin und/oder mindestens ein Phospholipid und/oder Squalan und/oder eine Fettkomponente umfasst.

5. Kosmetische Formulierung nach einem der vorangehenden Ansprüche,
**dadurch gekennzeichnet, dass**
das Verhältnis Polyol (z. B.) Glyzerin : Phospholipid(e) zwischen 100:1 und 1:1, vorzugsweise zwischen 30:1 und 5:1, insbesondere bei 15:1 liegt.

6. Kosmetische Formulierung nach einem der vorangehenden Ansprüche,
**dadurch gekennzeichnet, dass**
das Verhältnis Squalan : farblose(s) Karotinoid(e) zwischen 2000:1 und 100:1, vorzugsweise zwischen 1000:1 und 100:1, beträgt.

7. Kosmetische Formulierung nach einem der vorangehenden Ansprüche,
**dadurch gekennzeichnet, dass**
das Phospholipid einen Phosphatidylcholin-Gehalt (PC-Gehalt) zwischen 50% und 90 %, insbesondere zwischen 65%-75% aufweist.

8. Verfahren zur Herstellung der Formulierung nach einem der vorhergehenden Ansprüche, umfassend die Schritte:
a) Bereitstellen einer Mischung aus mindestens einem Polyol, aus mindestens einem Phospholipid, einer Fettkomponente, mindestens einem farblosen Karotinoid, wobei als farbloses Karotinoid Phytoen und/oder Phytofluen in nanomizellierter Form zum Einsatz kommt, sowie Glycosyl-Ceramiden und/oder Digalactosyl-Diglyzeriden,
b) Dispergieren dieser Mischung durch Rühren und
c) Weiterverarbeiten der Mischung durch Hochdruckhomogenisation zu einer insbesondere transparenten Mischung
d) Zusetzen einer wässrigen, farblosen antioxidativen Aminosäure unter Rühren.

9. Verfahren nach Anspruch 8 **dadurch gekennzeichnet, dass**
die Fettkomponente durch den Einsatz von Sonnenblumenöl und/oder anderer Triglyzeride enthaltene Öle und/oder Jojobaöl und/oder Sheabutter bereitgestellt wird.

## Claims

1. A cosmetic formulation for dermatological use, containing at least one carotenoid, wherein
- phytoene and/or phytofluene is present as the carotenoid, and wherein
- the phytoene and/or phytofluene is present in a nano-micellized form and comprises at least one water-soluble, antioxidative amino acid, the cosmetic formulation comprising glycosyl ceramides and/or digalactosyl diglycerides.

2. The cosmetic formulation according to claim 1,
wherein at least one water-soluble amino acid is ergothioneine.

3. The cosmetic formulation according to claim 1 or 2,
**characterized in that**
the water-soluble antioxidative amino acid is integrated in the nano-micellization of the carotenoids.

4. The cosmetic formulation according to any one of the preceding claims,
**characterized in that**
the cosmetic formulation furthermore comprises polyol, in particular glycerine and/or at least one phospholipid and/or squalene and/or a fatty component.

5. The cosmetic formulation according to any one of the preceding claims,
**characterized in that**
the ratio of polyol (e.g.) glycerine : phospholipid(s) is between 100:1 and 1:1, preferably between 30:1 and 5:1, in particular 15:1.

6. The cosmetic formulation according to any one of the preceding claims,
**characterized in that**
the ratio of squalene : colorless carotenoid(s) is between 2000:1 and 100:1, preferably between 1000:1 and 100:1.

7. The cosmetic formulation according to any one of the preceding claims,
**characterized in that**
the phospholipid has a phosphatidylcholine content (PC content) of between 50% and 90%, in particular of between 65%-75%.

8. A method for preparing the formulation according to any one of the preceding claims, comprising the steps of:
a) providing a mixture of at least one polyol, at least one phospholipid, a fatty component, at least one colorless carotenoid, wherein phytoene and/or phytofluene in a nano-micellized form is employed as the colorless carotenoid, and glycosyl ceramides and/or digalactosyl diglycerides,
b) dispersing said mixture by stirring, and
c) further processing the mixture by high-pressure homogenization into an in particular transparent mixture,
d) adding an aqueous, colorless water-soluble amino acid while stirring.

9. The method according to claim 1,
**characterized in that**
the fatty component is provided by using sunflower oil and/or oil containing other triglycerides and/or jojoba oil and/or shea butter.

## Revendications

1. Formulation cosmétique à usage dermatologique, contenant au moins une caroténoïde, sachant que
- du phytoène et/ou du phytofluène est présent comme caroténoïde et sachant que
- le phytoène et/ou le phytofluène est présent sous forme nanomicellisée et,
au moins un acide aminé antioxydant hydrosoluble, sachant que la formulation cosmétique comprend des céramides de glycosyl et/ou des diglycérides de digalactosyl.

2. Formulation cosmétique selon la revendication 1,
sachant qu'au moins un acide aminé hydrosoluble est de l'ergothionéine.

3. Formulation cosmétique selon la revendication 1 ou 2,
**caractérisée en ce que**
l'acide aminé antioxydant hydrosoluble est intégré dans la nanomicellisation de la caroténoïde.

4. Formulation cosmétique selon l'une des revendications précédentes,
**caractérisée en ce que**
la formulation cosmétique comprend en outre du polyol, en particulier de la glycérine et/ou au moins un phospholipide et/ou de la squalane et/ou un composant gras.

5. Formulation cosmétique selon l'une des revendications précédentes,
**caractérisée en ce que**
le rapport polyol (p. ex.) glycérine : phospholipide(s) est compris entre 100:1 et 1:1, de préférence entre 30:1 et 5:1, en particulier de 15:1.

6. Formulation cosmétique selon l'une des revendications précédentes,
**caractérisée en ce que**
le rapport squalane : caroténoïde(s) incolore(s) est compris entre 2000:1 et 100:1, de préférence entre 1000:1 et 100:1.

7. Formulation cosmétique selon l'une des revendications précédentes,
**caractérisée en ce que**
le phospholipide présente une teneur en phosphatidylcholine (teneur en PC) comprise entre 50% et 90%, en particulier entre 65% et 75%.

8. Procédé de fabrication de la formulation selon l'une des revendications précédentes, comprenant les étapes :
a) fourniture d'un mélange composé d'au moins un polyol, d'au moins un phospholipide, d'un composant gras, d'au moins une caroténoïde incolore, sachant que du phytoène et/ou du phytofluène sous forme nanomicellanisée est mis en oeuvre comme caroténoïde incolore, ainsi que des céramides de glycosyl et/ou des diglycérides de digalactosyl,
b) dispersion d'un mélange par agitation et
c) transformation ultérieure du mélange par homogénéisation à haute pression en un mélange en particulier transparent
d) addition d'un acide aminé aqueux antioxydant incolore moyennant agitation.

9. Procédé selon la revendication 8,
**caractérisé en ce que**
le composant gras est fourni par l'utilisation d'huile de tournesol et/ou d'autres huiles contenant des triglycérides et/ou de l'huile de jojoba et/ou du beurre de karité.
